# EUROPEAN PATENT SPECIFICATION

(11) **EP 1 786 796 B1**
(45) Date of publication and mention of the grant of the patent: **05.03.2008**
(21) Application number: 05784523.2
(22) Date of filing: 02.09.2005
(51) Int. Cl.: C07D 301/32, C07D 301/14

(54) **PROCESS**
VERFAHREN
PROCEDE

(30) Priority: 02.09.2004 SG 200406384
(43) Date of publication of application: 23.05.2007
(73) Proprietor: Shell Internationale Research Maatschappij B.V., 2596 HR Den Haag (NL)
(72) Inventor: NISBET, Timothy Michael, NL-1031 CM Amsterdam (NL); SNODGRASS, Kevin Michael, Singapore 627879 (SG)
(74) Representative: Zeestraten, Albertus W. J.
(86) International application number: PCT/EP2005/054320
(87) International publication number: WO 2006/024663

(56) References cited:
- EP-A- 0 345 856
- EP-A- 0 566 356
- EP-A- 0 569 248
- WO-A-20/04020369

## Description

The present invention relates to a process of manufacturing propylene oxide and styrene.

A commonly known process for manufacturing propylene oxide and styrene involves the steps of (i) reacting ethylbenzene with oxygen or air to form ethylbenzene hydroperoxide, (ii) reacting the ethylbenzene hydroperoxide thus obtained with propene in the presence of an epoxidation catalyst to yield propylene oxide and 1-phenylethanol, and (iii) converting the 1-phenylethanol into styrene by dehydration using a suitable dehydration catalyst. Such process has been described for example in EP-A-345856 and EP-A-0569248.

By-products are formed in the oxidation of ethylbenzene, in the reaction of ethylbenzene hydroperoxide with propene and in the dehydration of 1-phenylethanol into styrene. Well known by-products are methylphenylketone, acids such as benzoic acid and glycolic acid, 2-phenylethanol and dimers such as bis(α,α-phenyl ethyl)ether. The reaction mixtures generally are purified in order to remove the by-products.

A process conventionally applied comprises (a) contacting propene with a mixture of ethylbenzene hydroperoxide and ethylbenzene to obtain propylene oxide and 1-phenylethanol, (b) separating propylene oxide from the reaction mixture, (c) subjecting the mixture obtained in step (b) to one or more distillation steps to obtain a purified stream containing 1-phenylethanol, (d) contacting the purified stream containing 1-phenylethanol with a catalyst to obtain styrene and water, (e) removing styrene and water from the reaction mixture obtained in step (d). The remainder of the reaction mixture obtained in step (e) or part of this remainder, can be recycled to step (d) if such stream contains precursors for styrene.

Conventionally, streams containing precursors for styrene are recycled by combining them in step (c) with a stream containing 1-phenylethanol. A difficulty is that these precursors containing streams additionally contain undesired by-products. Recycle of such streams tends to give an undesirable build-up of by-products. It was further found that the by-product 2-phenylethanol is especially difficult to separate from 1-phenylethanol.

It has now been found that by-products such as 2-phenylethanol can be removed more efficiently if streams containing precursors for styrene are recycled in a specific way.

Therefore, the present invention relates to a process of manufacturing propylene oxide and styrene which process comprises:
(i) contacting propene with a mixture of ethylbenzene hydroperoxide and ethylbenzene in the presence of catalyst to obtain propylene oxide and 1-phenylethanol,
(ii) separating propylene oxide from the reaction mixture obtained in step (i),
(iii) subjecting the mixture obtained in step (ii) to distillation to obtain a top stream containing ethylbenzene and a bottom stream containing 1-phenylethanol,
(iv) subjecting the bottom stream containing 1-phenylethanol obtained in step (iii) to a further distillation to obtain a top stream containing purified 1-phenylethanol and a bottom stream containing heavy by-products,
(v) contacting the top stream containing 1-phenylethanol obtained in step (iv) with a dehydration catalyst to obtain styrene and water,
(vi) subjecting the reaction mixture obtained in step (v) to distillation to obtain a top stream containing styrene and water and a bottom stream containing unconverted 1-phenylethanol, 2-phenylethanol, methylphenylketone and by-products,
(vii) subjecting the bottom stream containing unconverted 1-phenylethanol, 2-phenylethanol, methylphenylketone and further by-products obtained in step (vi) to distillation to obtain a top stream containing methylphenylketone and a bottom stream containing 2-phenylethanol and heavy by-products, and
(viii) recycling to step (iv) the bottom stream containing 2-phenylethanol and heavy by-products obtained in step (vii) wherein the bottom stream obtained in step (vii) is introduced into the distillation of step (iv) below the point at which the bottom stream containing 1-phenylethanol obtained in step (iii) is introduced.

Although the option has not been mentioned explicitly, either all or part of the top streams and bottoms streams can be treated. Generally, it is preferred to treat substantially all of the top and/or bottom streams in order to make efficient use of the process.

The expressions top stream and bottom stream have been used for ease of reference. However, these expressions should not be interpreted narrowly. The top stream will be recovered from the higher part of the distillation column while the bottom stream will be recovered from the lower part.

The expression distillation as used in the present invention covers any separation of components based on a difference in volatility. Generally, the distillation will be carried out with the help of a fractionating tower. However, flash distillation also is suitable in many process steps. In order to improve the separation, the distillation can be carried out in the presence of an inert gas, so-called stripping.

In the process of the present invention, the bottom stream obtained in step (vii) is introduced into the distillation of step (iv) below the point at which the bottom stream obtained in step (iii) is introduced. The distillation of step (iv) can consist of one or more distillation columns. If 2 or more distillation columns are present, there is a subsequent column which is fed with the bottom stream of the column at which the bottom stream obtained in step (iii) is introduced. In this set-up, introduction of the bottom stream obtained in step (vii) at the subsequent column also is introduction of the bottom stream of step (vii) below the point at which the bottom stream of step (iii) is introduced. If the streams are added to a single fractionating tower, the bottom stream obtained in step (vii) is added to a stage below the stage in which the bottom stream of step (iii) is added.

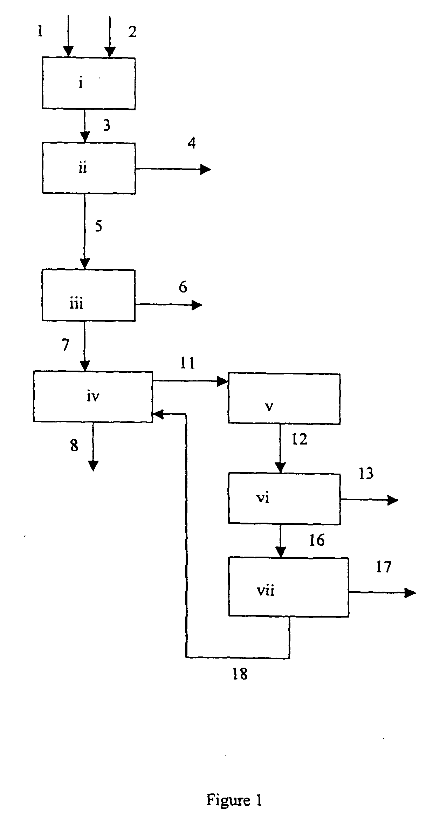

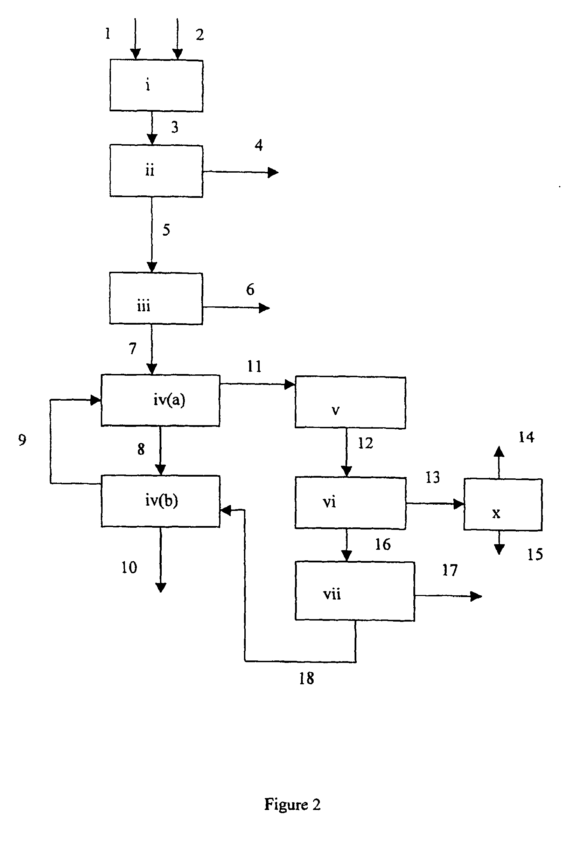

Process set-ups which can be used have been depicted schematically in Figures 1 and 2.

Figure 1 shows a process in which the bottom stream containing 1-phenylethanol obtained in step (iii) is subjected to distillation in a single column.

Figure 2 shows a process in which the bottom stream containing 1-phenylethanol obtained in step (iii) is subjected to distillation in 2 columns.

The process according to the present invention is discussed further with the help of these Figures. The processes of Figures 1 and 2 differ only in process step (iv) and in the further treatment of stream 13.

Ethylbenzene hydroperoxide and propene are added to the process of the present invention as streams 1 and 2. In step (i), the ethylbenzene hydroperoxide is contacted with propene to yield propylene oxide and 1-phenylethanol in the presence of a catalyst.

Generally, this process step (i) will further comprise separating unconverted propene from the reaction mixture obtained. The unconverted propene which is removed subsequently can be recycled to step (i). Separating unconverted propene has the advantage that a smaller amount of reaction mixture is to be treated further.

The effluent from the epoxidation step (i) (stream 3) is subsequently subjected to a separation treatment in step (ii) to remove the propylene oxide formed (stream 4). Such separation can be done in any way known to the person skilled in the art. Preferably, the propylene oxide is separated by distillation from the reaction mixture obtained in step (i).

After the separation of step (ii), part or all of the remainder of the reaction mixture (stream 5) is subjected to a distillation in step (iii) in which ethylbenzene is separated from 1-phenylethanol. The ethylbenzene containing stream 6 can be used again in an earlier stage of the process such as in the manufacture of ethylbenzene hydroperoxide by oxidation of ethylbenzene.

In the process of Figure 1, the bottom stream containing 1-phenylethanol obtained in step (iii)(stream 7) is subjected to a further distillation in a single distillation column in step (iv) to obtain a top stream 11 and a bottom stream 8. The latter contains heavy by-products. Stream 8 can be used in a process as described in a process as described in EP-A-1056697, more specifically the process in which a residual fraction obtained in the dehydration of 1-phenylethanol is first subjected to a separation treatment to remove methylphenylketone and to a further separation treatment together with effluent from a preceding epoxidation step to remove 1-phenylethanol or substituted 1-phenylethanol before the residual fraction thus obtained is subjected to a cracking treatment. Alternatively, stream 8 can be sent to a process for treating waste streams such as a furnace.

In the process of Figure 2, stream 7 is subjected to a distillation in 2 separate columns. The distillation treatments in the separate columns have been given the numbers iv(a) and iv(b). Stream 7 is subjected to distillation in column (iv)(a) to obtain a top stream 11 which is sent to process step (v) and a bottom stream 8 which is sent to distillation column (iv)(b). The distillation in column (iv)(b) gives a top stream 9 and a bottom stream 10. The bottom stream 10 is removed from the process and can be used in a process as described in EP-A-1056697. Alternatively, stream 10 can be sent to a process for treating waste streams such as a furnace. Top stream 9 contains 1-phenylethanol and methylphenylketone and is recycled to distillation step iv(a), preferably to the bottom of the distillation column.

The processes of Figures 1 and 2 will be discussed together again hereinafter.

The top stream 11 obtained in step (iv) is contacted with a dehydration catalyst to obtain a reaction mixture containing styrene, water and further compounds (stream 12).

The reaction mixture obtained in step (v) (stream 12) is subsequently subjected to distillation in step (vi) to obtain a stream 13 containing styrene and water.

Preferably, the present process further comprises separating styrene from the top stream containing styrene and water obtained in step (vi) to obtain purified styrene as depicted in Figure 2.

The remainder of the reaction mixture obtained in step (iv) (stream 16) contains unconverted 1-phenylethanol, 2-phenylethanol, methylphenylketone and by-products. This stream 16 is separated further by distillation in step (vii) to obtain a top stream 17 mainly containing methylphenylketone. This top stream usually also contains a certain amount of 1-phenylethanol. The bottom stream 18 contains 2-phenylethanol and heavy by-products. This bottom stream usually also contains a certain amount of 1-phenylethanol. The latter stream 18 is recycled to the distillation column employed in step (iv).

The epoxidation step (i) and the dehydration step (v) are well known.

A homogeneous catalyst or a heterogeneous catalyst can be applied in the epoxidation step. Molybdenum compounds are frequently applied as homogeneous catalysts. The epoxidation step is preferably carried out with a heterogeneous catalyst. The catalyst may comprise as the catalytically active metal one or more transition metals, such as vanadium, molybdenum, tungsten, titanium and zirconium. One particularly suitable class of epoxidation catalysts are the titanium-based catalysts. The titanium can be present as the metal per se or in any other form such as titania and titanium containing salts. Furthermore, it has been found particularly advantageous to use catalysts containing titanium on a silicon containing carrier. A suitable silicon containing carrier is silica. Examples of such catalysts are for instance described in US-A-4,367,342 and EP-A-0,345,856.

Suitable process conditions comprise an average temperature in the epoxidation reactor of from 50 to 150 °C, preferably of from 60 to 135 °C. The pressure in each reactor can be up to 80 bar, preferably of from 10 to 60 bar. Generally, the reaction medium is in the liquid phase.

Dehydration catalysts are well known in the art. Both a homogeneous and a heterogeneous catalyst can be used in the present process. Preferably, a heterogeneous dehydration catalyst is employed. Suitable dehydration catalysts include for instance acidic materials like alumina, alkali alumina, aluminium silicates and H-type synthetic zeolites. Dehydration conditions are also well known and usually include reaction temperatures of 150-250 °C for liquid phase dehydration and 210-320 °C, typically 280-310 °C, for gas phase dehydration. Pressures usually range from 0.1 to 10 bar. In principle any known dehydration process can be applied in the process according to the present invention. For the purpose of the present invention gas phase dehydration is preferred. In a preferred embodiment the gas phase dehydration is carried out at a temperature in the range of 250 to 320 °C using an alumina-based dehydration catalyst.

The distillation of step (iv) preferably is carried out in 2 distillation columns. In this set-up, it is preferred that the first distillation is carried out with the help of a reboiler. The latter means that part of the bottom fraction is removed, heated with steam or a hot process stream, and subsequently sent back to the distillation column. The second distillation generally will be carried out in the presence of stripping gas, preferably steam.

Styrene is to be removed from the top stream containing styrene and water obtained in step (vi) to obtain purified styrene. Separation is preferably carried out by phase separation of the mixture of styrene and water into an organic phase and an aqueous phase, followed by subjecting the organic phase containing styrene to one or more distillations.

Process step (vii) gives a top stream containing methylphenylketone. Additionally, this top stream usually contains a certain amount of 1-phenylethanol. In a preferred embodiment, the top stream containing methylphenylketone obtained in step (vii) is subjected to hydrogenation to obtain a reaction mixture containing 1-phenylethanol which is subsequently recycled to step (iii).

The invention is further illustrated by the following examples without restricting its scope to these particular embodiments.

### Example according to the invention

Calculations were carried out on a process according to the schedule as depicted in Figure 2 and described further hereinbelow.

A mixture containing 20 %wt of ethylbenzene hydroperoxide, 40 %wt of propene and 40 %wt of ethylbenzene was contacted with a catalyst as described in the Example of EP-A-345856 at a temperature of 90 °C and a pressure of 40 x 10⁵ N/m².

Unconverted propene and propylene oxide were separated by distillation from the reaction mixture obtained. The remainder of the product was subjected to a further distillation to obtain a top stream containing ethylbenzene and a bottom stream containing 1-phenylethanol.

The bottom stream containing 1-phenylethanol was subjected to the distillation discussed in detail above for Figure 2. The distillation produced a top stream containing purified 1-phenylethanol and a bottom stream containing heavy by-products. The bottom stream was removed from the process.

The top stream containing 1-phenylethanol obtained in the distillation of the bottom stream containing 1-phenylethanol, was contacted with a trilobe-shaped alumina catalyst at a pressure of 1 x 10⁵ N/m² and 300 °C to obtain styrene. The alumina catalyst had a surface area of 110 m²/g, a pore volume of 0.77 ml/g and a particle diameter of 2.5 mm.

The reaction mixture obtained in the dehydration of 1-phenylethanol was treated further according to Figure 2.

The removal of 2-phenylethanol and the losses of the styrene precursors 1-phenyl ethanol and methyl phenyl ketone in stream 10 were calculated. The operating parameters and relevant flows in distillation step (iv) are given in Table 1. Distillation stages are numbered from the top (stage 1) of the columns. Stream 9 passed as vapour from distillation step iv(b) to step iv(a).

It can be seen that 80 kg/h of 2-phenylethanol is removed in stream 10, whereas only 8 kg/h in total of the styrene precursors 1-phenylethanol and methyl phenyl ketone are removed in stream 10.

### Comparative Example

The process described in the Example according to the invention, was repeated with the difference that the bottom stream containing 2-phenylethanol and heavy by-products obtained in step (vii) was combined with the bottom stream containing 1-phenylethanol obtained in step (iii). The combined streams were sent to the first column of the distillation of step (iv).

The operating parameters and relevant flows in distillation step (iv) are again given in Table 1. The condenser duty, the reboiler duty and the amount of steam added were the same in the exemplified process according to the invention and the comparative one.

It can be seen that in this comparative process only 70 kg/h of 2-phenylethanol is removed in stream 10, whereas 8 kg/h in total of the styrene precursors 1-phenylethanol and methyl phenyl ketone are removed in stream 10.

**Table 1**

| | Example according to the invention | Comparative example |
|---|---|---|
| Step iv(a) | | |
| | | |
| Top pressure (x 10⁵ N/m²) | 1.9 | 1.9 |
| Number of stages | 25 | 25 |
| Feed tray stream 7 | 14 | 14 |
| Feed tray stream 18 | 23 | 14 |
| Temperature stage 1 (°C) | 215 | 215 |
| Temperature stage 25 (°C) | 239 | 239 |
| | | |
| Total flow stream 7 (kg/h) | 15788 | 15830 |
| 1-phenylethanol in stream 7 (kg/h) | 13331 | 13326 |
| methylphenylketone in stream 7 (kg/h) | 1960 | 1961 |
| 2-phenylethanol in stream 7 (kg/h) | 177 | 202 |
| | | |
| Total flow stream 18 (kg/h) | 1184 | 1255 |
| 1-phenylethanol in stream 18 (kg/h) | 46 | 49 |
| methylphenylketone in stream 18 (kg/h) | 243 | 243 |
| 2-phenylethanol in stream 18 (kg/h) | 62 | 82 |

| Step iv(b) | | |
|---|---|---|
| | | |
| Number of stages | 10 | 10 |
| Feed tray stream 8 | 1 | 1 |
| Temperature stage 1 (°C) | 187 | 187 |
| Temperature stage 25 (°C) | 176 | 176 |
| | | |
| Total flow stream 10 (kg/h) | 1044 | 1045 |
| 1-phenylethanol in stream 10 (kg/h) | 7.5 | 7.7 |
| methylphenylketone in stream 10 (kg/h) | 0.4 | 0.1 |
| 2-phenylethanol in stream 10 (kg/h) | 80 | 70 |

## Claims

1. Process of manufacturing propylene oxide and styrene which process comprises:
(i) contacting propene with a mixture of ethylbenzene hydroperoxide and ethylbenzene in the presence of catalyst to obtain propylene oxide and 1-phenylethanol,
(ii) separating propylene oxide from the reaction mixture obtained in step (i),
(iii) subjecting the mixture obtained in step (ii) to distillation to obtain a top stream containing ethylbenzene and a bottom stream containing 1-phenylethanol,
(iv) subjecting the bottom stream containing 1-phenylethanol obtained in step (iii) to a further distillation to obtain a top stream containing purified 1-phenylethanol and a bottom stream containing heavy by-products,
(v) contacting the top stream containing 1-phenylethanol obtained in step (iv) with a dehydration catalyst to obtain styrene and water,
(vi) subjecting the reaction mixture obtained in step (v) to distillation to obtain a top stream containing styrene and water and a bottom stream containing unconverted 1-phenylethanol, 2-phenylethanol, methylphenylketone and by-products,
(vii) subjecting the bottom stream containing unconverted 1-phenylethanol, 2-phenylethanol, methylphenylketone and further by-products obtained in step (vi) to distillation to obtain a top stream containing methylphenylketone and a bottom stream containing 2-phenylethanol and heavy by-products, and
(viii) recycling to step (iv) the bottom stream containing 2-phenylethanol and heavy by-products obtained in step (vii), wherein the bottom stream obtained in step (vii) is introduced into the distillation of step (iv) below the point at which the bottom stream containing 1-phenylethanol obtained in step (iii) is introduced.

2. Process according to claim 1, in which process the distillation of step (iv) is carried out in 2 distillation columns.

3. Process according to claim 2, in which process the first distillation of step (iv) is carried out with the help of a reboiler.

4. Process according to claim 2 and/or 3, in which process the second distillation of step (iv) is carried out in the presence of stripping gas.

5. Process according to any one of the preceding claims, in which process the top stream containing methylphenylketone obtained in step (vii) is subjected to hydrogenation to obtain a reaction mixture containing 1-phenylethanol which is subsequently recycled to step (iii).

6. Process according to any one of the preceding claims, which process further comprises separating styrene from the top stream containing styrene and water obtained in step (vi) to obtain purified styrene.

## Patentansprüche

1. Verfahren zur Herstellung von Propylenoxid und Styrol, welches Verfahren umfasst:
(i) das Inkontaktbringen von Propen mit einem Gemisch aus Ethylbenzolhydroperoxid und Ethylbenzol in Gegenwart eines Katalysators, um Propylenoxid und 1-Phenylethanol zu erhalten,
(ii) das Abtrennen des Propylenoxids aus dem in Schritt (i) erhaltenen Reaktionsgemisch,
(iii) das Unterwerfen des im Schritt (ii) erhaltenen Gemisches unter eine Destillation, um einen Ethylbenzol enthaltenden Kopfstrom und einen 1-Phenylethanol enthaltenden Sumpfstrom zu erhalten,
(iv) das Unterwerfen des 1-Phenylethanol enthaltenden Sumpfstroms, welcher im Schritt (iii) erhalten wird, unter eine weitere Destillation, um einen gereinigten, 1-Phenylethanol enthaltenden Kopfstrom und einen schwere Nebenprodukte enthaltenden Sumpfstrom zu erhalten,
(v) das Inkontaktbringen des 1-Phenylethanol enthaltenden Kopfstromes, welcher im Schritt (iv) erhalten wird, mit einem Dehydratisierungskatalysator, um Styrol und Wasser zu erhalten,
(vi) das Unterwerfen des im Schritt (v) erhaltenen Reaktionsgemisches, unter eine Destillation, um einen Styrol und Wasser enthaltenden Kopfstrom und einen Sumpfstrom zu erhalten, welcher nicht umgewandelten 1-Phenylethanol, 2-Phenylethanol, Methylphenylketon und Nebenprodukte enthält,
(vii) das Unterwerfen des Sumpfstromes, welcher nicht umgewandelten 1-Phenylethanol, 2-Phenylethanol, Methylphenylketon und weitere Nebenprodukte enthält, der im Schritt (vi) erhalten wird, unter eine Destillation, um einen Methylphenylketon enthaltenden Kopfstrom und einen 2-Phenylethanol und schwere Nebenprodukte enthaltenden Sumpfstrom zu erhalten, und
(viii) das Recyclieren des 2-Phenylethanol und schwere Nebenprodukte enthaltenden Sumpfstroms, welcher im Schritt (vii) erhalten wird, in den Schritt (iv), wobei der im Schritt (vii) erhaltene Sumpfstrom in die Destillation von Schritt (iv) unterhalb des Punktes eingebracht wird, an welchem der 1-Phenylethanol enthaltende Sumpfstrom, welcher im Schritt (iii) erhalten wird, eingeführt wird.

2. Verfahren nach Anspruch 1, in welchem Verfahren die Destillation von Schritt (iv) in zwei Destillationskolonnen ausgeführt wird.

3. Verfahren nach Anspruch 2, in welchem Verfahren die erste Destillation von Schritt (iv) mit Hilfe eines Verdampfers ausgeführt wird.

4. Verfahren nach Anspruch 2 und/oder 3, in welchem Verfahren die zweite Destillation von Schritt (iv) in Gegenwart von Stripgas ausgeführt wird.

5. Verfahren nach einem der vorstehenden Ansprüche, in welchem Verfahren der Methylphenylketon enthaltende Kopfstrom, welcher im Schritt (vii) erhalten wird, einer Hydrierung unterworfen wird, um ein 1-Phenylethanol enthaltendes Reaktionsgemisch zu erhalten, welches darauffolgend in den Schritt (iii) recycliert wird.

6. Verfahren nach einem der vorstehenden Ansprüche, welches Verfahren ferner das Abtrennen von Styrol aus dem Styrol und Wasser enthaltenden Kopfstrom, welcher im Schritt (vi) erhalten wird, umfasst, um gereinigtes Styrol zu erhalten.

## Revendications

1. Procédé de fabrication d'oxyde de propylène et de styrène, un tel procédé consistant :
(i) A mettre en contact du propène avec un mélange d'hydropéroxyde d'éthylbenzène en présence de catalyseur pour obtenir de l'oxyde de propylène et du 1-phényléthanol,
(ii) à séparer l'oxyde de propylène du mélange réactif obtenu à l'étape (i),
(iii) à soumettre le mélange obtenu à l'étape (ii) à la distillation pour obtenir un flux de tête contenant de l'éthylbenzène et un flux de fond contenant 1-phényléthanol,
(iv) à soumettre le flux de fond contenant le 1-phényléthanol obtenu à l'étape (iii) à une autre distillation pour obtenir un flux de tête contenant le 1-phényléthanol purifié et un flux de fond contenant des sous-produits lourds,
(v) à mettre en contact le flux de tête contenant le 1-phényléthanol obtenu à l'étape (iv) avec un catalyseur de déshydratation pour obtenir du styrène et de l'eau,
(vi) à soumettre le mélange réactif obtenu à l'étape (v) à la distillation pour obtenir un flux de tête contenant du styrène et de l'eau et un flux de fond contenant 1-phényléthanol, 2-phényléthanol, du méthylphénylcéton et des sous produits,
(vii) à soumettre le flux de fond contenant 1-phényléthanol, 2-phényléthanol, du méthylphénylcétone et des autres sous-produits obtenus à l'étape (vi) à la distillation pour obtenir un flux de tête contenant du méthylphénylcétone et un flux de fond contenant 2-phényléthanol et des sous-produits lourds, et
(viii) à recycler à l'étape (iv) le flux de fond contenant 2-phényléthanol et les sous-produits lourds obtenus à l'étape (vii), le flux de fond obtenu à l'étape (vii) étant introduit dans la distillation de l'étape (iv) en-dessous du point auquel le flux de fond contenant le 1-phényléthanol obtenu à l'étape (iii) est introduit.

2. Procédé selon la revendication 1, dans lequel procédé la distillation de l'étape (iv) est menée dans deux colonnes de distillation.

3. Procédé selon la revendication 2, dans lequel procédé la première distillation de l'étape (iv) est menée avec l'aide d'un rebouilleur.

4. Procédé selon la revendication 2 et/ou 3, dans lequel procédé la seconde distillation de l'étape (iv) est menée en présence d'un gaz de stripage.

5. Procédé selon n'importe laquelle des revendications précédentes, dans lequel procédé le flux de tête contenant du méthylphénylcétone obtenu à l'étape (vii) est soumis à hydrogénation pour obtenir un mélange de réaction contenant 1-phényléthanol, lequel est par la suite recyclé à l'étape (iii)

6. Procédé selon n'importe laquelle des revendications précédentes, lequel procédé comprend en outre la séparation du styrène du flux de tête contenant du styrène et de l'eau obtenu à l'étape (vi) pour obtenir du styrène purifié.
